(19) Europäisches Patentamt
European Patent Office
Office européen des brevets

(11) EP 1 315 832 B1

(12) **EUROPÄISCHE PATENTSCHRIFT**

(45) Veröffentlichungstag und Bekanntmachung des Hinweises auf die Patenterteilung:
**08.06.2005 Patentblatt 2005/23**

(21) Anmeldenummer: **01967309.4**

(22) Anmeldetag: **04.09.2001**

(51) Int Cl.[7]: **C12Q 1/527**

(86) Internationale Anmeldenummer:
**PCT/EP2001/010151**

(87) Internationale Veröffentlichungsnummer:
**WO 2002/020831 (14.03.2002 Gazette 2002/11)**

(54) **VERFAHREN ZUR MESSUNG DER AKTIVITÄT DER NO-SYNTHASE**

METHOD FOR MEASUREMENT OF THE ACTIVITY OF NO-SYNTHASE

PROCEDE POUR MESURER L'ACTIVITE DE LA NO SYNTHASE

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **06.09.2000 DE 10043845**

(43) Veröffentlichungstag der Anmeldung:
**04.06.2003 Patentblatt 2003/23**

(73) Patentinhaber: **Grünenthal GmbH**
**52078 Aachen (DE)**

(72) Erfinder:
- **HENNIES, Hagen-Heinrich**
  **52152 Simmerath (DE)**
- **SUNDERMANN, Bernd**
  **52066 Aachen (DE)**

(56) Entgegenhaltungen:
- **KNOWLES RICHARD G ET AL: "Measurement of NOS activity by conversion of radiolabeled arginine to citrulline using ion-exchange separation." METHODS IN MOLECULAR BIOLOGY, Bd. 100, 1998, Seiten 67-73, XP008007063 1998 Humana Press Inc. Suite 808, 999 Riverview Drive, Totowa, New Jersey 07512, USA ISBN: 0-89603-537-9**
- **BREDT D S ET AL: "ISOLATION OF NITRIC OXIDE SYNTHETASE A CALMODULIN-REQUIRING ENZYME" PROCEEDINGS OF THE NATIONAL ACADEMY OF SCIENCES OF THE UNITED STATES, Bd. 87, Nr. 2, 1990, Seiten 682-685, XP002210297 1990 ISSN: 0027-8424 in der Anmeldung erwähnt**
- **BREDT D S ET AL: "NITRIC OXIDE MEDIATES GLUTAMATE-LINKED ENHANCEMENT OF CYCLIC GMP LEVELS IN THE CEREBELLUM" PROCEEDINGS OF THE NATIONAL ACADEMY OF SCIENCES OF THE UNITED STATES, Bd. 86, Nr. 22, 1989, Seiten 9030-9033, XP002210298 1989 ISSN: 0027-8424**
- **CUNNINGHAM J M ET AL: "Interleukin-1-beta-mediated inhibition of arginase in RINm5F cells." CYTOKINE, Bd. 9, Nr. 8, 1997, Seiten 570-576, XP002213216 ISSN: 1043-4666**

EP 1 315 832 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft ein Verfahren zur Messung der Aktivität der NO-Synthase, ein entsprechendes Verfahren zur Identifizierung von NO-Synthase-Modulatoren, insbesondere zum Einsatz in einem HTS (high throughput screening), und die Verwendung der entsprechenden Kationenaustausch-Filterplattenmembranen.

Es gehört seit über 100 Jahren zum bekannten medizinischen Wissen, Nitroglycerin zur Behandlung koronarer Herzkrankheiten zu verwenden. Man hat allerdings erst vor 15 Jahren erkannt, daß diese Wirkung auf die Bildung von Stickoxid (NO) zurückzuführen ist. Seitdem ist die Bedeutung dieses Moleküls an einer ständig wachsenden Zahl von Publikationen ablesbar. Insbesondere in pharmakologischer Hinsicht sind daher die Synthesewege des NO und insbesondere die Auffindung die NO-Synthese modulierender Verbindungen von großer Bedeutung.

NO wird durch die NO-Synthasen (EC: 1.14.13.39) gebildet (im weiteren manchmal als NOS abgekürzt). Diese katalysieren die Oxidation von L-Arginin zu L-Citrullin und NO über $N^G$-Hydroxyarginin. Die NO-Synthasen haben in der monomeren Form eine Molekülmasse zwischen 125 und 155 kDa, sind jedoch nur als Homodimer aktiv. Die molekulare Struktur der NO-Synthasen ähnelt stark derjenigen der Cytochrom-P450-Reduktasen. Es sind verschiedene Klassen von NO-Synthasen bekannt:

- die durch Cytokine oder LPS induzierbare NOS = iNOS und
- die durch $Ca^{2+}$ aktivierte konstitutive cNOS, die weiter in die folgenden Unterformen unterteilt wird:
  - endotheliale NOS = eNOS
  - neuronale NOS = nNOS.

In der Literatur gibt es zahlreiche Beispiele, wie man die Aktivität der bekannten NOS-Isoenzyme bestimmen kann.

Am bekanntesten sind folgende Verfahren:

1.) Abtrennung des Endproduktes Citrullin mit Hilfe der Säulenchromatographie

$$[^3H]\text{L-Arginin} \xrightarrow[\text{NADPH}]{\text{NOS}} [^3H]\text{L-Citrullin} + \text{NO}$$

Die Enzymansätze werden nach beendeter Reaktion über separate Kationenaustauschersäulen gegeben. Danach erfolgt Elution des $[^3H]$L-Citrullin und Bestimmung der Eluate am β-counter. Der Nachteil dieser Methode liegt darin, daß pro Enzymansatz eine separate Säulenchromatographie stattfinden muß (z.B. pro 96er Mikrotiterplatte (MTP) braucht man 96 Chromatographiesäulen). Methode nach: D.S. Bredt u. S.H. Snyder, Proc. Natl. Acad. Sci. **87,** 682 (1990).

2.) $NO_2$-Bestimmung mit dem "Griess-Reagenz"

Neben L-Citrullin fällt bei der NOS-Reaktion (s. Reaktion 1.)) auch NO als Endprodukt an, das jedoch instabil ist und somit weiterreagiert:

$$2\,NO + O_2 \rightarrow 2\,NO_2$$

$$2\,NO_2 + H_2O \rightarrow NO_2^- + NO_3^- + 2H^+$$

Das entstandene $NO_3^-$ muß zu $NO_2^-$ reduziert werden. Dieses kann mit Cadmium oder enzymatisch durch Zugabe von Nitratreduktase geschehen. Da überschüssiges NADPH aus dem NOS-Ansatz die Griess-Reaktion mit $NO_2^-$ stört, müssen diese Reduktionsäquivalente entfernt werden. Das kann enzymatisch geschehen, indem Lactatdehydrogenase/Pyruvat hinzugegeben wird. Nach Zugabe des Griess-Reagenzes (1 % Sulfanilamid; 0,1 % Naphthylendiamin-Dihydrochlorid; 5 % $H_3PO_4$) entsteht ein purpurfarbener AZO-Farbstoff, der bei 543 nm bestimmt werden

kann.

Die Griesssche Reaktion läuft wie folgt ab:

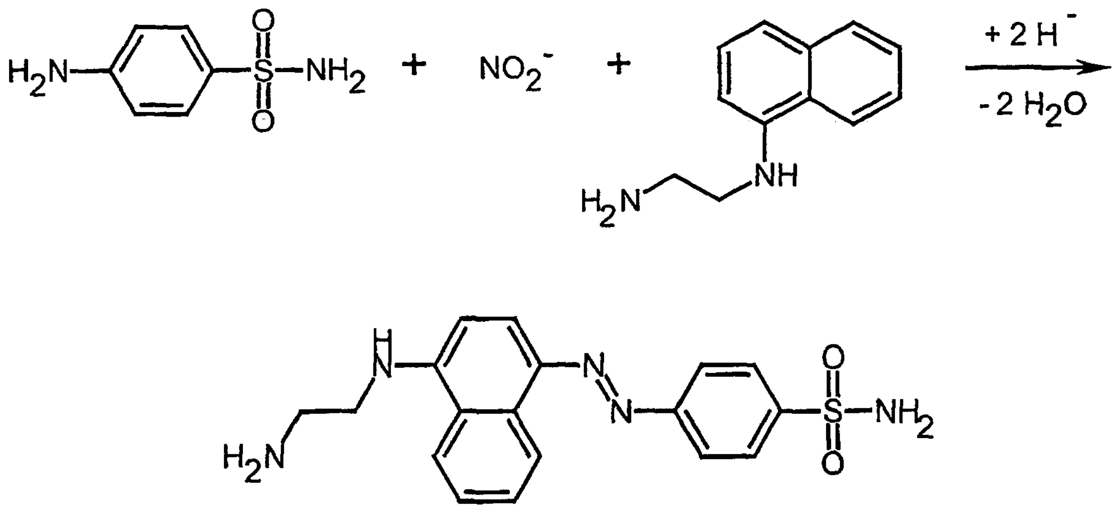


Literatur: L.C. Green, D.A. Wagner, J. Glogowski, P.L. Skipper, J.S. Wishnok, S.R. Tannenbaum, Anal. Biochem. **126**, 131 (1982)

Der Nachteil dieser Meßmethode ist, daß zur Bestimmung der NOS-Aktivität mehrere Reaktionsschritte nötig sind, die getrennt nacheinander durchgeführt werden müssen.

3.) Chemilumineszenzmessung:

Das neben L-Citrullin bei der NOS-Reaktion anfallende NO reagiert zu $NO_2^-$ und $NO_3^-$ (s. Reaktion 2)). Für die Chemilumineszenzmessung müssen $NO_2^-$ und $NO_3^-$ aber zu NO rücküberführt werden. Dann wird eine Reaktion mit Ozon durchgeführt:

$$NO + O_3 \rightarrow NO_2^* + O_2$$

$$NO_2^* \rightarrow NO_2 + hv$$

Literatur: S. Archer, FASEB Journal **7,** 349 (1993)

Der Nachteil dieser Methode ist, daß zum einen auch hier mehrere Reaktionsschritte vorliegen, zum anderen Ozon wegen der geringen Halbwertzeit für die Reaktion aufwendig direkt hergestellt werden muß.

4.) Bestimmung von cGMP mit Hilfe der durch NO stimulierbaren Guanylatcyclase:

Dabei wird aus der Menge an cGMP auf die Aktivität der Guanylatcyclase geschlossen. Da die Guanylatcyclase durch NO aktiviert wird, kann so auf die entstandene Menge an NO, die Aktivität der NOS, zurückgeschlossen werden.

Literatur: M. Feelisch, E:a: Noack, Eur. J. Pharmacol. **139,** 19 (1987) Mayer, Schmidt, Humbert, Böhme, Biochem. Biophys. Research Commun. **164,** 678 (1989)

Der Nachteil dieser Methode ist, daß es sich um eine gekoppelte Enzymreaktion handelt. Abgesehen davon, daß gekoppelte Enzymreaktionen die Messung einer Anfangsgeschwindigkeit meist nicht erlauben, müssen die Substrate für eine saubere lineare Bestimmung der Reaktionsgeschwindigkeit in Konzentrationen im Sättigungsbereich der Enzyme vorliegen (~ 100 $K_m$). Das kann ein erheblicher Kostenfaktor sein. Bei dieser Reaktion ist im übrigen auch

die Aktivität der Guanylatcyclase nicht konstant, da sie erst durch während der gekoppelten Reaktion gebildetes NO stimuliert wird.

Aufgrund der Notwendigkeit, in der modernen Pharmaforschung eine erhebliche Anzahl von Messungen innerhalb kürzester Zeit durchzuführen, insbesondere auch große Mengen verschiedener Substanzen, sog. Libraries, auf einzelne interessante Substanzen mit möglicher physiologischer Wirkung hin zu untersuchen, werden sog. HTS-Verfahren (High-Throughput-Screening-Verfahren) durchgeführt. Dabei werden Testverfahren eingesetzt, die automatisierbar, möglichst einfach und schnell sind und daher insbesondere mit einem Minimum an einfachen automatisierbaren Verfahrensschritten und ohne manuelle Zwischenschritte auskommen. Es genügt ein "grobes Verfahren", das nur eine ja-nein Antwort liefern muß. Insofern sind Messungen im Bereich der linearen Anfangsgeschwindigkeit einer Enzymreaktion nicht unbedingt einzuhalten. Zeitfressende Manipulationen wie Entnahme von aliquoten Teilmengen, weitere Inkubationen durch gekoppelte (Enzym)reaktionen, Zentrifugationsschritte, die nicht automatisierbar sind etc., scheiden für einen HTS-Betrieb von vornherein aus. Gleichzeitig müssen die Tests aber im Ergebnis zuverlässig sein. Daher ist für die Durchführung eines HTS-Laufes die Präzision des Assays von entscheidender Bedeutung, d.h. das "signal to noise ratio" sollte möglichst hoch sein, damit nicht die Präzision der Aussage durch eine doppel-, dreifach- oder gar vierfach-Bestimmung erkauft werden muß, was wiederum einem hohen Substanzdurchsatz entgegensteht.

Alle vorstehend aufgeführten Reaktionen 1) - 4) sind aber nicht HTS-kompatibel, da hier gekoppelte Enzymreaktionen, Entnahme von aliquoten Teilen, Zentrifugationsschritte, säulenchromatographische Trennschnitte etc. einem high throughtput screening entgegenstehen. Der Aufwand an Zeit bzw. Verfahrensschritten, um die nötige Genauigkeit zu erzielen, ist für ein HTS-Verfahren zu erheblich, und die Verfahren sind weitgehend nicht automatisierbar.

Aufgabe der vorliegenden Erfindung war es daher, ein vereinfachtes Verfahren zur Messung der Aktivität der NO-Synthase, insbesondere auch ein Verfahren zur Identifizierung von NO-Synthase-Modulatoren, zu entwickeln. Insbesondere sollte dieses Verfahren im HTS, dem High-Throughput-Screening, einsetzbar sein.

Diese Aufgabe wird durch ein Verfahren zur Messung der Aktivität der NO-Synthase mit folgenden Verfahrensschritten gelöst:

(a) Inkubation der NO-Synthase mit markiertem Arginin als Substrat in einem Reaktionsgefäß,
(b) Trennung des markierten Arginins von dem gegebenenfalls als Produkt der enzymatischen Reaktion entstandenen, markierten Citrullin, zu einem Zeitpunkt, zu dem die Konzentration an Citrullin ansteigt,
(c) Messung der Menge an jeweils abgetrenntem Arginin,

wobei die Trennung über eine Kationenaustausch-Filterplatten-Membran erfolgt.

Dabei versteht man im Sinne dieser Erfindung unter:

- Inkubation: das Einbringen und Belassen eines biologischen Objektes, hier eines Enzyms, NOS, in ein Medium, dem unter Umständen weitere Verbindungen zugesetzt werden oder worden sind wie z.B. das Enzymsubstrat. Dabei werden geeignete (meist den physiologischen nahe) Bedingungen gewählt, um einen bestimmten Effekt zu erreichen, hier den Ablauf einer Enzymreaktion, der durch NOS katalysierten Umwandlung von Arginin in Citrullin und NO. Die Bedingungen werden beispielsweise durch Kontrolle der Temperatur, des pH, der Coenzyme bzw. Cofaktoren und/oder der Reaktionszeit etc. erreicht.
- Reaktionsgefäß: Darunter ist ein Gefäß zu verstehen, in dem eine chemische Reaktion unter kontrollierten Bedingungen aber auch eine auf Bindung oder anderen physikalischen Kräften beruhende Trennung ablaufen kann, beispielsweise ein Eppendorf-Cup, eine Kulturschale oder -flasche, ein Reagenzglas, ein Zentrifugenröhrchen oder ein "Well" einer Mikrotiterplatte. Dabei kann das Reaktionsgefäß auch durch selektiv durchlässige Membranen in Kompartimente unterteilt sein und/oder muß insbesondere nicht zwangsläufig flüssigkeitsundurchlässig verschlossen sein, auch nicht auf der Unterseite des Gefäßes. Damit können Reaktionsgefäße auch an einer Seite (meist der Unterseite) mit einer selektiv durchlässigen Membran, beispielsweise einer Filterplattenmembran, verschlossene Säulchen oder Mikrotiterplatten sein. In einem Reaktionsgefäß findet die Inkubation (Schritt a)) wie auch vorzugsweise die Trennung (Schritt b)) statt.
- Trennung: Damit ist die örtliche Trennung des Arginin vom Citrullin gemeint, so daß - im Gegensatz zu dem beide Substanzen homogen in einem bestimmten Verhältnis enthaltenden Inkubationsansatz - separate Bereiche entstehen, wobei in einem Bereich das Arginin fast vollständig konzentriert und das Citrullin im Verhältnis zur vorherigen Konzentration fast vollständig verschwunden ist und es sich in einem anderen Bereich umgekehrt verhält. Die Trennung kann z.B. physikalisch über Größen- bzw. Molekulargewichtsfilter, über die Ladung (Kationenaustauscher, chromatographisch oder durch Anlegung einer Spannung) oder z.B. durch Zentrifugation erfolgen. Im erfindungsgemäßen Fall erfolgt die Trennung über eine Filterplattenmembran.
- markiert: Unter Markierung versteht man die Messbarmachung eines Moleküls, beispielsweise durch Kopplung an ein anderes Molekül, das hilft, ein Meßsignal zu generieren (z.B. Fluoreszenz; Lumineszenz, Metallion), oder durch Verwendung von radioaktiven Atomen bei dessen Synthese (radioaktive Markierung).

- Zeitpunkt, zu dem die Konzentration an Citrullin ansteigt: Damit ist ein Zeitpunkt gemeint, bei dem die Reaktion noch in Richtung auf das Produkt Citrullin läuft, also kein Endpunkt erreicht ist.
- Messung: Unter einer Messung ist in diesem Sinne die Quantifizierung der markierten Substanz mit dem geeigneten Gerät zu verstehen.
- Filterplattenmembran: Das ist eine flache Membran, also eine definiert durchlässige Grenzfläche, die in der Lage ist, Flüssigkeiten zu filtern, also bestimmte Inhaltsstoffe der Flüssigkeit zurückzuhalten.

Hier wurde als Verfahren eine Trennung zwischen Substrat und Endprodukt durch Einsatz einer Filterplatten-Membran gewählt, wobei durch einen einfachen Filtrationsschritt eine quantitative Separation zwischen Substrat und den Endprodukten stattfindet, ohne daß einzelne Elutionsfraktionen aufgefangen werden müssen. Durch diesen simplen "Filtrationsschritt" wird eine hohe Präzision erlangt, wobei eine Doppel- oder Dreifach-Bestimmung überflüssig wird. Erst die Einführung dieser Filterplatten-Membran erlaubt den erfolgreichen Einsatz des Verfahrens beispielsweise in einem HTS, aber auch ganz allgemein für einfache unkomplizierte Bestimmungen der NOS-Aktivität. Als Substrat besonders bevorzugt ist markiertes L-Arginin, was u.a. zur Bildung von L-Citrullin als einem Produkt führt.

Außerdem erbrachte ein Vergleich der IC50-Wert-Bestimmungen verschiedener bekannter NOS-Inhibitoren nach der Methode nach Bredt u. Snyder (1990), der unter Nummer 1 bei Würdigung des Standes der Technik aufgeführte "Abtrennung des Endproduktes Citrullin mit Hilfe der Säulenchromatographie", und nach einem erfindungsgemäßen Verfahren, daß auch in dieser Hinsicht das erfindungsgemäße Verfahren trotz seines einfachen Aufbaus präzise und valide ist und qualitativ überraschenderweise mit der aufwendigen Standardmethode nach Bredt u. Snyder (1990) durchaus konkurrieren kann.

Es ist besonders bevorzugt, wenn das Verfahren so ausgeführt wird, daß in mindestens einem der Reaktionsgefässe dem Inkubationsansatz in Schritt (a) eine Substanz zugefügt wird, die daraufhin überprüft werden soll, ob sie ein Modulator, insbesondere Aktivator oder Inhibitor, vorzugsweise Inhibitor, der NO-Synthase ist. Damit ist der Einsatz in einem Screening-Assay umfaßt, mit dem Substanzen auf physiologische Wirksamkeit untersucht werden. Dabei versteht man unter Modulator ein Molekül, das das Verhalten - hier der NOS - beeinflußt, insbesondere zu einer erhöhten Aktivität (Aktivator) oder einer erniedrigten Aktivität (Inhibitor) bis hin zur vollständigen Blockade führt.

Insbesondere ist auch Gegenstand der Erfindung ein Verfahren zur Identifizierung von NO-Synthase-Modulatoren mit folgenden Verfahrensschritten:

(a) Inkubation einer zu testenden Substanz unter geeigneten Bedingungen mit NO-Synthase und markiertem Arginin in einem Reaktionsgefäß,
(b) Trennung des markierten Arginins von dem gegebenenfalls als Produkt der enzymatischen Reaktion entstandenen, markierten Citrullin, zu einem Zeitpunkt, zu dem die Konzentration an Citrullin ansteigt,
(c) Messung der Menge an jeweils abgetrenntem Arginin,

wobei die Trennung über eine Kationenaustausch-Filterplatten-Membran erfolgt.

Dabei versteht man unter "einer zu testenden Substanz" insbesondere eine niedermolekulare Verbindung, deren Wirkung auf die NOS-Aktivität untersucht werden soll.

Der Gegenstand ist damit insbesondere ein Screeningverfahren, mit dem, beispielsweise aus einer Library, Substanzen identifiziert werden können, die Modulatoren der NOS und damit potentiell pharmakologisch interessante Verbindungen sind. Dabei versteht man unter "einer zu testenden Substanz" insbesondere niedermolekulare Verbindungen, deren Wirkung auf die NOS-Aktivität untersucht werden soll. Es ist dabei besonders bevorzugt, wenn das Verfahren zur Identifizierung eines Aktivators oder Inhibitors, vorzugsweise eines Inhibitors, der NO-Synthase dient.

Weiter wird in einer bevorzugten Ausführungsform der erfindungsgemäßen Verfahren bei der Trennung gemäß Schritt (b) aus dem Reaktionsgefäß das Inkubationsmedium entfernt. Mit Inkubationsmedium ist das Medium gemeint, in dem die Inkubation gemäß Schritt (a) stattfindet und in dem das Substrat (markiertes Arginin), NOS, die entstehenden Produkte (also markiertes Citruilin und NO) und deren Folgeprodukte sowie Coenzyme bzw. Cofaktoren gelöst sind und das mit diesen gelösten oder evt. suspendierten Substanzen zusammmen den Inkubationsansatz nach Schritt (a) bildet. Dabei ist es besonders bevorzugt, wenn das Inkubationsmedium weitgehend, insbesondere fast vollständig, vorzugsweise vollständig, entfernt wird. Damit ist insbesondere auch eine Entfernung über die Kationenaustausch-Filterplattenmembran gemeint, wobei weitgehend heißt, daß nur noch geringe Tropfmengen des Mediums verbleiben, fast vollständig heißt, daß nur die Kationenaustausch-Filterplattenmembran geringe Mengen an Inkubationsmedium enthält und vollständig heißt, daß das Medium entweder aus der Kationenaustausch-Filterplattenmembran ausgewaschen oder durch Trocknung entfernt wurde. Es ist weiter bevorzugt, daß bei der Entfernung des Inkubationsmediums das markierte Arginin von dem Inkubationsmedium getrennt wird. Unter den Folgeprodukten im Sinne dieser Erfindung versteht man aus den Produkten hervorgehende weitere Produkte, z.B. insbesondere $NO_2^-$ oder $NO_3^-$. Ein Coenzym ist eine niedermolekulare, nicht proteinogene Verbindung, die als ein nur vorübergehend u. locker an ein Enzym gebundenes Co-Substrat benötigt wird, z.B.: NADPH. Cofaktoren ist eine Sammelbezeichnung für niedermolekulare Substanzen, deren Anwesenheit für enzymatische Reaktionen erforderlich ist oder sein kann, z.B.: $Ca^{2+}$, Calmodulin.

Im Zusammenhang mit der Trennung nach Schritt (b) ist es weiter bevorzugt, wenn bei der Trennung gemäß Schritt (b) zusätzlich Cofaktoren bzw. Coenzyme, die NO-Synthase, NO und die Folgeprodukte und/oder die NO-Synthase von dem markierten Arginin getrennt werden. Damit ist die Abtrennung der dem Inkubationsmedium im Inkubationsansatz nach Schritt (a) zugegebenen Substanzen außer dem Substrat gemeint. Da diese Substanzen meist wasserlöslich sind, fällt dies überwiegend mit der Entfernung des Inkubationsmediums zusammen. Kationenaustauscher sind Stoffe, die im Austausch äquivalente Mengen fremder

Kationen aus Flüssigkeiten aufnehmen. Die Filterplatten-Membranen können weiter bevorzugt Papierfilter oder Textilfilter sein oder aus sonstigen porösen oder durchbrochenen Festmaterialien, beispielsweise Metall, Glas oder Keramik, bestehen oder diese enthalten. Unter ersterem werden Filter auf Papier- oder Textilbasis verstanden. Mit letzterem sind z.B. unbeschichtete oder mit z.B. Gel oder Harz beschichtete Metallfoliensiebe(-filter) oder Ton- bzw. Glasfilter gemeint. Weiter bevorzugt ist es auch, wenn die funktionellen Gruppen für den Kationenaustausch an den Filterplatten-Membranen fixiert sind. Dabei sind funktionelle Gruppen negative Gruppen, die als Bindungsstelle für die Kationen dienen und an den Träger, hier die Filterplatten-Membranen, gebunden sind, beispielsweise kovalent aber evt. auch über starke Ionenbindungen.

Bevorzugte funktionelle Gruppen, die in den im erfindungsgemäßen Verfahren eingesetzten Kationenaustauscher-Filterplattenmembranen verwendet werden, sind Phosphat-, Carbonat-, Phosphonat-, Sulfat- und/oder Sulfonat-Gruppen, vorzugsweise Phosphat-Gruppen.

Dabei weisen die Filterplatten-Membranen, wenn es sich um Papierfilter handelt, insbesondere eine Dicke von 10,00 mm bis 0,10 mm, vorzugsweise 1,00 mm bis 0,15 mm, insbesondere 0,50 mm bis 0,20 mm, vorzugsweise 0,23 mm auf. Weiter bevorzugt ist es auch, wenn die Filterplatten-Membranen Kationenaustauscher mit einer Austauschkapazität von $\geq 1{,}0\ \mu Eq./cm^2$, insbesondere $\geq 10{,}0\ \mu Eq./cm^2$, vorzugsweise $\geq 18{,}0\ \mu Eq./cm^2$ sind. Dabei können diese Filterplatten eine Durchflußgeschwindigkeit von > 100 mm/ 30 min, vorzugsweise 125 mm/30 min haben.

In einer besonders bevorzugten Ausführungsform der erfindungsgemäßen Verfahren erfolgt Schritt (a) parallel in mehreren Reaktionsgefäßen annähernd gleichzeitig, vorzugsweise gleichzeitig, und/oder Schritt (b) parallel in mehreren Reaktionsgefäßen annähernd gleichzeitig, vorzugsweise gleichzeitig, insbesondere bezüglich des Inkubationsbeginns in Schritt (a) und/oder dem Beginn der Trennung nach Schritt (b). Dabei ist es besonders bevorzugt, daß die Reaktionsgefäße zusammenhängen, es sich insbesondere um Wells einer Mikrotiterplatte handelt. Dabei versteht man unter "Well" die die Inkubationsansätze oder Proben aufnehmenden Vertiefungen in einer Mikrotiterplatte. Als Mikrotiterplatten, die in den erfindungsgemäßen Verfahren eingesetzt werden können, sind besonders solche geeignet, die < 96 "Wells", vorzugsweise 24 oder 48 "Wells", oder ≥ 96 "Wells", insbesondere 96, 384, 864 oder 1536 "Wells", aufweisen.

Weiter ist es bevorzugt, wenn die in dem erfindungsgemäßen Verfahren eingesetzten Reaktionsgefäße für Schritt (a) und/oder Schritt (b) ein Volumen zur Aufnahme von ≤ 2 ml, insbesondere ≤ 1 ml, vorzugsweise ≤ 1ml, ≤ 800 µl, ≤ 350 µl, ≤ 300 µl, ≤ 250 µl, < 200 µl, ≤ 150 µl, ≤ 100 µl, ≤ 50 µl, ≤ 30 µl oder ≤ 5 µl aufweisen.

In einer besonders bevorzugten Ausführungsform der erfindungsgemäßen Verfahren erfolgt die Abtrennung gemäß Schritt (b) durch Absaugen des Inkubationsmediums aus Schritt (a) durch die Filterplatten-Membran. Dabei wird ein Unterdruck auf der dem Inkubationsansatz abgewandten Seite der Filterplattenmembran angelegt. Mit Inkubationsmedium ist das Medium gemeint, in dem die Inkubation gemäß Schritt (a) stattfindet und in dem Substrat, NOS, Produkte und Folgeprodukte sowie Coenzyme bzw. Cofaktoren gelöst sind. In einer weiteren, besonders bevorzugten Ausführungsform der erfindungsgemäßen Verfahren wird nach der Abtrennung gemäß Schritt (b) die Fraktion, die das vom markierten Arginin getrennte markierte Citrullin enthält, verworfen. Dabei ist mit Fraktion eine Teilmenge des vor der Trennung bestehenden Inkubationsansatzes gemeint, in diesem Falle Inkubationsmedium mit bestimmten gelösten Anteilen. Hier ist das der Teil des Mediums, in dem das markierte Citrullin des Inkubationsansatzes weitgehend vollständig und das markierte Arginin des ursprünglichen Inkubationsansatzes (fast) nicht mehr vorliegt. Gerade diese beiden letzten Ausführungsformen erleichtern die Handhabung und Automatisierung des Tests weiter erheblich und machen ihn besonders geeignet für ein HTS.

In einem weiteren bevorzugten erfindungsgemäßen Verfahren liegt das nach der Abtrennung gemäß Schritt (b) abgetrennte Substrat auf oder in der Filterplattenmembran vor, wobei es vorzugsweise gebunden ist.

Besonders bevorzugt ist es, wenn die Markierung die Messung des Substrats über Radioaktivität, Fluoreszenz, Lumineszenz oder Spektrums- bzw. Farbveränderung erlaubt. In einer besonders bevorzugten Ausführungsform erfolgt die Messung des Substrats im Szintillationszähler nach Zugabe von Szintillationsflüssigkeit auf die Filterplatten-Membran. Damit ist insbesondere gemeint, daß in einem besonders bevorzugten Verfahrensschritt nach der Trennung gemäß Schritt (b) die Filterplattenmembran, auf der das markierte Arginin vorliegt, entweder einzeln oder als ganze mehrere Filterplattenmembranen umfassende Mikrotiterplatte getrocknet wird, beispielsweise im Trockenschrank. Mikrotiterplatten (MTP) werden anschließend auf der Rückseite versiegelt, einzelne Filtermembranen hingegen in Szintillationsgefäße überführt. Dann werden die Filtermembranen mit Szintillationsflüssigkeit übergossen, abgeschlossen

(MTP auf der Vorderseite versiegelt) und in einem Counter, einem Szintillationszähler, gezählt.

In einer weiteren Variante des Verfahrens finden Schritt (a) und Schritt (b) nicht im gleichen Reaktionsgefäß statt. Auch diese Variante ist besonders gut für ein HTS geeignet. Damit ist insbesondere gemeint, daß der Inkubationsansatz gemäß Schritt (a) in ein weiteres Gefäß überführt wird, das beispielsweise die Filterplattenmembran enthält und in dem Schritt (b) stattfindet. So werden beispielsweise parallel die Inkubationsansätze aus einer Mikrotiterplatte in eine andere Mikrotiterplatte überführt, deren Wells am Boden eine Filterplattenmembran; aufweisen, die durch ein flüssigkeitsdurchlässiges Sieb fixiert wird. Hier kann dann beispielsweise die Trennung über das Anlegen eines Unterdrucks auf der Unterseite der Filterplattenmembran erfolgen, wobei das Inkubationsmedium mit z.B. dem markierten Citrullin zusammen abgesaugt wird und das markierte Arginin auf oder in der Filterplattenmembran am Boden der Wells verbleibt.

Eine weitere besonders interessante Variante des erfindungsgemäßen Verfahrens sieht vor, daß nach der Abtrennung gemäß Schritt (b) ein Waschschritt erfolgt, vorzugsweise ≤ 3 mal, insbesondere 1 mal, vorzugsweise mit Wasser oder Puffer insbesondere mit einem Volumen ≤ 10 ml, insbesondere ≤ 5 ml, vorzugsweise ≤ 3 ml, insbesondere ≤ 2 ml. Das Volumen der Waschflüssigkeit bezieht sich auf die Menge pro getrenntem (Schritt b) Inkubationsansatz gemäß (Schritt a), vorzugsweise also pro Reaktionsgefäß. Auch dies kann den Einsatz im HTS verbessern und insbesondere eine größere Genauigkeit des Tests erlauben. Dabei kann das Waschvolumen deutlich höher sein als das Volumen der Reaktionsgefäße, sofern parallel zur Zugabe der Waschflüssigkeit diese wieder, beispielsweise durch Absaugen, entfernt wird. Wenn dies nicht der Fall ist, entspricht das Volumen der Waschfraktionen vorzugsweise dem maximalen des Reaktionsgefäßes, in dem Schritt (b) durchgeführt wird.

Eine bevorzugte Ausführungsform des Verfahrens sieht vor, daß als NO-Synthase rekombinante NO-Synthase verwendet wird, wobei die NO-Synthase insbesondere subtypspezifisch ist, vorzugsweise eine human-, ratten- oder maus-spezifische eNOS, iNOS oder nNOS, insbesondere auch α- bzw. β-nNOS ist. Dabei versteht man unter rekombinanter NOS eine NOS, die unter Verwendung einer gentechnologischen Manipulation hergestellt ist. Beispielsweise wird ausgehend von der Sequenz eines klonierten NOS-Gens ein rekombinantes DNA-Konstrukt, beispielsweise ein Klonierungs- oder besser Expressionsvektor, konstruiert. Mit dem wird dann eine Zelle transfiziert, die anschließend unter besonderen Kulturbedingungen das Gen exprimiert und das Protein, rekombinante NOS, bildet. Dabei bedeuten:

- <u>(rekombinantes) DNA-Konstrukt:</u> Generelle Bezeichnung für jede Art von DNA-Molekülen, die durch die in vitro-Verknüpfung von DNA-Molekülen entstanden sind.

- <u>Klonierungsvektor:</u> Generelle Bezeichnung für Nukleinsäure-Moleküle, die beim Klonieren als Träger von Fremdgenen oder Teilen dieser Gene dienen.

- <u>Expressionsvektor:</u> Bezeichnung für speziell konstruierte Klonierungsvektoren, die nach Einbringen in eine geeignete Wirtszelle die Transkription und Translation des in den Vektor einklonierten Fremdgens erlauben.

Es ist aber besonders bevorzugt, wenn die beim erfindungsgemäßen Verfahren eingesetzte NO-Synthase Teil eines Rohextrakts (ab hier manchmal NOS-Rohextrakt genannt) aus Wirbeltier-, vorzugsweise Säugetier-, Gewebe ist. Dabei kann der Rohextrakt aus Nagetieren gewonnen werden, und/oder aus dem ZNS und/oder er kann der aus Homogenat gewonnene Überstand sein, insbesondere der Überstand eines Homogenats aus Ratten- oder Maus-Cerebellum. Dabei wird dem ZNS (zentralen Nervensystem) eines Nagetiers, vorzugsweise der Ratte oder Maus, Gewebe, vorzugsweise das Cerebellum, entnommen, dieses homogenisiert, zentrifugiert und der Überstand als neuronaler NOS-Rohextrakt verwendet. Ein Beispiel für ein entsprechendes Herstellungsverfahren findet sich in den Beispielen.

Es ist weiter bevorzugt, daß die eingesetzte Proteinmenge im Inkubationsansatz im Verfahren gemäß Schritt (a) pro $mm^2$ der in Schritt (b) eingesetzten Filterplattenmembran maximal ≤ 5,0 µg, insbesondere ≤ 2,5 µg, vorzugsweise ≤ 1,0 µg, sein darf. Die angegebenen Proteinmengen sind Erfahrungswerte, oberhalb von denen die Filterplattenmembran bei der Trennung, insbesondere beim Absaugen, verstopfen kann.

Gereinigte NOS (auch z.B. rekombinantes Enzym) benötigt neben dem Substrat und Coenzym NADPH ferner noch weitere Coenzyme/Cofaktoren wie Tetrahydrobiopterin ($BH_4$), FAD, FMN, Calmodulin (CaM) und $Ca^{++}$ (A.J. Hobbs, A. Higgs, S. Moncada, Annu. Rev. Pharmacol. Toxicol. <u>**39,**</u> 191, 1999). Daher ist es zunächst bevorzugt, wenn bei einem erfindungsgemäßen Verfahren dem Inkubationsansatz gemäß Schritt a) Cofaktoren bzw. Coenzyme und/oder NADPH hinzugefügt werden. Insbesondere können dem Inkubationsansatz Calmodulin, $Ca^{2+}$, Tetrahydrobiopterin, FAD und/oder FMN hinzugefügt werden.

Um die Kosten bei einer HTS-Kampagne so gering wie möglich zu halten, kann man alternativ auch mit dem bereits erwähnten NOS-Rohextrakt arbeiten, wobei auf die Zugabe eines großen Teils oben erwähnter Coenzyme verzichtet werden kann. Das heißt, daß bei Verwendung des oben erwähnten neuronalen NOS-Rohextraktes auf den Einsatz von $BH_4$, CaM, FAD und FMN verzichtet werden kann. Daher ist es besonders bevorzugt, wenn bei Verwendung

des oben beschriebenen NOS-Rohextraktes dem Inkubationsansatz NADPH und $Ca^{2+}$, insbesondere als einzige Cofaktoren bzw. Coenzyme, hinzugefügt werden. Gerade diese Variante ist besonders kostengünstig.

Es ist weiter bevorzugt, daß beim erfindungsgemäßen Verfahren der Inkubationsansatz in Puffer als Inkubationsmedium angesetzt wird, und ebenso, daß die Inkubation in Schritt (a) bei Raumtemperatur, 37° C, 25° C, 10° C oder 4° C, vorzugsweise Raumtemperatur, stattfindet.

Weiter ist es besonders bevorzugt, wenn die Trennung gemäß Schritt (b) 1- 600 min, insbesondere 3 - 120 min, vorzugsweise 5 - 60 min nach Beginn der Inkubation gemäß Schritt (a) stattfindet. Diese Zeiten hängen insbesondere von der Aktivität der eingesetzten NOS ab.

Eine besonders bevorzugte Form des erfindungsgemäßen Verfahrens ist für ein HTS (High-Throughput-Screening) geeignet, d.h. insbesondere einfach, reproduzierbar und auch gut automatisierbar.

Ein entscheidendes Kriterium für die Qualität eines Testverfahrens (Assays), insbesondere für ein HTS, ist das "signal-to-noise-ratio", das Verhältnis zwischen den niedrigen Meßsignalen und dem durchschnittlichen Hintergrundrauschen (ohne Signal). Dort sollte ein besonders großes Verhältnis angestrebt werden. Dies trifft auf die erfindungsgemäßen Verfahren zu. Diese zeigen, insbesondere in bevorzugten Ausführungsformen, ein "signal-to-noise-ratio" von $\geq 4$, insbesondere $\geq 5$, vorzugsweise $\geq 6$ oder $\geq 10$.

Dabei ist es besonders bevorzugt, wenn das Verfahren der Messsung der Aktivität der NO-Synthase und/ oder der Identifizierung von NO-Synthase-Modulatoren, insbesondere Aktivatoren oder Inhibitoren dient, insbesondere, wenn es sich um ein erfindungsgemäßes Verfahren handelt, ganz besonders, wenn es sich bei den Verfahren um ein Verfahren im Rahmen eines HTS (high-throughput-screening) handelt.

Die folgenden Beispiele und Abbildungen sollen die Erfindung erläutern, ohne daß der Gegenstand der Erfindung darauf beschränkt wäre.

**Abbildungen und Beispiele**

**Abbildungen**

Figur 1) zeigt das graphisch aufbereitete Ergebnis eines Screening-Assays nach Beispiel 4 und entspricht der Tabelle 3. Dabei entsprechen x- und y-Achse der alphanummerischen Einteilung einer 96- well-Mikrotiterplatte und die z-Achse den gemessenen "corrected counts per minute" (ccpm), den um Nullwert und Quenchfaktor korrigierten, im Counter gezählten radioaktiven Zerfällen pro Minute.

**Beispiel 1:**

**Allgemeine Verfahrensweise für einen HTS-NOS-Assay**

In einem HTS-NOS-Assay wird radioaktives Arginin als Substrat benutzt. Das Assayvolumen kann je nach Art der Mikrotiterplatte (MTP) im Bereich zwischen 25 µl und 250 µl gewählt werden. In Abhängigkeit von der benutzten Enzymquelle werden Cofaktoren und Coenzyme zugefügt. Die Inkubation der Ansätze in dieser Mikrotiterplatte (Assay-MTP) gemäß Schritt (a) wird bei Raumtemperatur vorgenommen und beträgt je nach verwendeter Enzymaktivität (units) zwischen 5 und 60 Minuten. Zum Ende der Inkubation (Schritt (a)) wird die Platte in einen Zellharvester plaziert, der mit einer MTP bestückt ist, die eine Kationenaustauschermembran als Filterboden besitzt (Filter-MTP). Alle Ansätze der Assay-MTP werden in diese Filter-MTP überführt und über eine Kationenaustauscher-Filter-Platte, einen mit Phosphatgruppen beladenen Papierfilter, abgesaugt. Die Filter-MTP wird anschließend mit Puffer oder Wasser gewaschen. Mit Hilfe dieser Vorgehensweise wird das verbliebene Substrat Arginin auf dem Kationenaustauscher gebunden, während das enzymatisch gebildete radioaktive Citrullin quantitativ ausgewaschen wird. Nach Trocknen der Filter-MTP und Zugabe von Szintillationsflüssigkeit kann das gebundene Arginin am Szintillationszähler ausgezählt werden. Eine nicht gehemmte NOS-Reaktion spiegelt sich in einer geringen Radioaktivität wieder. Eine gehemmte Enzymreaktion bedeutet, daß das radioaktive Arginin nicht umgesetzt worden ist. Das heißt, auf dem Filter befindet sich eine hohe Radioaktivität.

Der Assay kann mit sehr geringen Substratkonzentrationen gefahren werden (z.B. 50 nM). Auch unter diesen Bedingungen liegt das "signal to noise ratio" noch bei über 6. Bei Verwendung höherer Substratkonzentrationen kann ein Verhältnis von mehr als 10 erzielt werden. Bedingt durch dieses extrem einfache Verfahren (der Inhalt einer Assay-MTP wird nur über eine Kationenaustauscher enthaltende Filterplatte abgesaugt (in der Filter-MTP), deren Radioaktivität dann gemessen wird), können je nach vorhandener HTS-Anlage mehrere 100-tausend Verbindungen einer Library in nur wenigen Tagen gescreent werden.

**Beispiel 2:**

**Beispiele verwendeter Materialien**

- Arginin, L-[2, 3, 4-$^3$H]-monohydrochlorid; Best.-Nr. NET-1123, NEN
- $CaCl_2$ wasserfrei; Best.- Nr. 2388.1000; Merck
- 1.4-Dithiothreitol (DTT), Best.-Nr. 708984; ROCHE
- $Na_2$EDTA-Dihydrat; Best.-Nr. 03680; FLUKA
- HEPES, Best:-Nr. H-3375; SIGMA
- NADPH, tetrasodium salt; Best.-Nr. 1585363; ROCHE
- TRIS; BEST.-Nr. 93349; FLUKA

| | |
|---|---|
| Enzym-Präparationspuffer | 50 mM Tris-HCl mit 1 mM EDTA: Der pH des Puffers wird bei 4 °C auf 7,4 eingestellt. |
| Inkubationspuffer (-medium) | 50 mM HEPES mit 1 mM EDTA; 1,25 mM $CaCl_2$ und 1 mM Dithiothreitol. Der pH des Puffers wird bei 25 °C auf 7,4 eingestellt. |
| Waschmedium | $H_2O$ |

**Beispiel 3:**

**Enzympräparation**

Als Ausgangsgewebe werden Ratten-Cerebelli benutzt. Pro Rattenkleinhim wird 1 ml Enzympräparationspuffer (4 °C) hinzugegeben. Die Tiere werden betäubt und getötet, das Gehirngewebe, das Cerebellum, wird herauspräpariert und mit einem Polytron-Homogenisierer für 1 min bei 6 000 U/min aufgeschlossen.

↓

Zentrifugation bei 4 °C für 15 min bei 20 000 g

↓

Überstand abdekantieren und bei -80°C portioniert einfrieren (Niederschieg verwerfen).

Der nNOS-haltige Gewebeüberstand ist unter diesen Lagerungsbedingungen lange stabil, sollte aber nur einmal aufgetaut und nicht wieder eingefroren werden.

**Beispiel 4:**

**Beispiel für einen HTS-NOS-Assay gemäß Beispiel 1 bis 3**

a) Inkubationsansatz (Schritt(a))

Verwendet werden 96-well MTP mit einer "Well"-Kapazität von ≤ 250 µl Pipettierreihenfolge, siehe Tabelle 1:

Tabelle 1:

| Substanz | Molarität i.A. | µl | * Protein i.A: |
|---|---|---|---|
| Inkubat.-Puffer | - | 100 | - |
| Testsubstanz | variabel; vorzugsweise $10^{-5}$M | variabel; vorzugsweise 20 µl | - |
| NADPH | 0,5 mM | 20 | - |
| Enzym (s. Beispiel 3) | - | variabel; maximales Volumen der Enzymlösung = 50 µl | variabel; maximale einsetzbare Proteinmenge = 100 µg |

* Proteinbestimmung nach O.H. Lowry et al; J. Biol.Chem. 193, 265 (1951) i.A. = im Ansatz

Tabelle 1: (fortgesetzt)

| Substanz | Molarität i.A. | µl | * Protein i.A: |
|---|---|---|---|
| [$^3$H]Substrat | variabel; vorzugsweise 50 nM | variabel; vorzugsweise 10 µl | - |
| Endvolumen: | | max. 250 µl | |

* Proteinbestimmung nach O.H. Lowry et al; J. Biol.Chem. **193**, 265 (1951) i.A. = im Ansatz

Nach beendetem Pipettiervorgang wird ein Deckel auf diese MTP (Assay-MTP) gelegt. Inkubation bei 25 °C, was der Raumtemperatur (RT) entspricht, für 5 - 60 min, je nach Menge und Aktivität des eingesetzten Enzyms.

b) Absaugschritt (Schritt (b))

Anschließend wird der Inhalt der Assay-MTP z.B. mit Hilfe eines 96-well Cell-Harvesters in eine 96-well Kationenaustauscher MTP (Filter-MTP) transferiert und abgesaugt. Es schließt sich eine einmalige Wäsche mit 200 m) $H_2O$ (aus einer Wanne) an.

c) Meßschritt (Schritt (c))

Dann wird die Platte für 1 h bei 60 °C im Trockenschrank getrocknet. Nun wird die Bodenseite der Filter-MTP von unten her exakt mit einem "back seal" versiegelt. Danach werden pro well 35 µl Szintillator hinzupipettiert. Ferner wird nunmehr die Plattenoberseite mit einem "top seal" versiegelt. Nach 1 h Wartezeit kann die Platte am β-counter ausgemessen werden (Messung kann auch sehr viel später erfolgen, da das Meßsignal stabil bleibt).

Im HTS-Betrieb empfiehlt es sich, das Inkubationsmedium, NADPH- und Enzymlösung vor Beginn des Pipettierschrittes zu vereinen, um nicht drei separate Pipettierungen vornehmen zu müssen, die zeitaufwendiger sind. Das Assay-Volumen kann bei gleichen Konzentrationen auch verkleinert werden.

**Beispiel 5:**

**Einsatz eines Verfahrens gemäß Beispiel 2 - 4 im Screening:**

Verwendet wurden:

Tabelle 2:

| | |
|---|---|
| Serocluster® | Mikrotiter-Platte für die Inkubation (Assay-MTP):<br>Firma Costar®, 96 Well, Polypropylene, Round Bottom, Kat. Nr. 3794 (Corning Costar Corporation, Cambridge, MA, USA) |
| Unifilter® | Kationenaustauscher-Filter-Platte in Mikrotiterplatte (Filter-MTP): Firma Whatman®, 96 Wells, 150 µl, Mesh Bottom. P81 Cellulose Phosphate Paper, Kat. Nr. 7700-0512<br>(Whatman Inc., Clifton, NJ 07014, USA)<br>Physikalische Eigenschaften:<br>Austauschkapazität: 18,0 µEq. / $cm^2$<br>Durchflußgeschwindigkeit: 125 mm/30 min<br>Dicke: 0,23 mm |
| Brandel® | 96-well Cell Harvester, Typ MPXRI-96T<br>(Brandel, Gaithersburg, MD 20877, USA) |
| Wallac® | Trilux 1450 Microbeta, Liquid Scintillation and Luminescence Counter (Wallac-ADL-GmbH, D-79111 Freiburg, Deutschland) |
| Enzym | Präparation aus Ratten-Cerebelli, s. Beispiel 3 |
| TopSeal™ | Firma Packard (Packard, Meriden, CT 06450, USA) |
| BackSeal | Firma Packard (Packard, Meriden, CT 06450, USA) |
| Szintillator | Typ: Ultima Gold™ (Packard, Meriden, CT 06450, USA) |

| | |
|---|---|
| Testsubstanzen | 1 x $10^{-5}$M im Ansatz |
| Interner Standard | 7-Nitroindazol, 1 x $10^{-5}$M im Ansatz |
| NADPH | 0,5 mM im Ansatz |
| [$^3$H] Substrat | 50 nM im Ansatz |
| Inkubationszeit | 30 min bei Raumtemperatur |

Alle anderen Parameter und Verfahrensschritte wie zuvor aufgeführt (siehe Beispiel 4).

Die Tabelle 3 zeigt eine Versuchsserie, in der 80 Substanzen getestet worden sind (Positionen A2-A11; B2-B11; C2-C11; D2-D11; E2-E11; F2-F11; G2-G11 und H2-H11). Dabei entspricht die alphanummerische Einteilung der einer 96- well-Mikrotiterplatte und es werden die gemessenen "corrected counts per minute" ccpm, die um Nullwert und Quenchfaktor korrigierten, im Counter gezählten radioaktiven Zerfälle pro Minute, angegeben.

Komplette Ansätze, jedoch ohne Enzym, geben den 100% [$^3$H]Arginin-Ausgangsgehalt an ("Max" = Positionen A1; B1; C1; A12; B12; C12).

Komplette Ansätze (mit Enzym) zeigen den Umsatz zu [$^3$H]Citrullin und NO dadurch an, daß der Gehalt an [$^3$H]Arginin abgenommen hat ("Min" = minimaler [$^3$H]Arginin-Gehalt = ungehemmte Enzymreaktion; Positionen D1, E1; F1; D12; E12 und F12). Dies kann jedoch bei NOS-Aktivatoren übertroffen werden, da dort noch mehr Arginin umgesetzt wird als bei unmoduliertem Enzym, so daß noch weniger Radioaktivität auf dem Filter verbleibt.

Als interner Standard (Referenz) wurde der NOS-Inhibitor 7-Nitroindazol in einer Konzentration von 1 x $10^{-5}$ M verwendet ("Ref" = G1; H1; G12 und H12).

Tabelle 3 :

| HIT-Erkennung | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | MAX<br>20023 ccpm | Object 1<br>10243 ccpm | Object 9<br>5016 ccpm | Object 17<br>5226 ccpm | Object 25<br>4551 ccpm | Object 33<br>4447 ccpm | Object 41<br>4813 ccpm | Object 49<br>5938 ccpm | Object 57<br>4715 ccpm | Object 65<br>5577 ccpm | Object 73<br>3615 ccpm | MAX<br>19867 ccpm |
| B | MAX<br>19667 ccpm | Object 2<br>4395 ccpm | Object 10<br>4663 ccpm | Object 18<br>4328 ccpm | Object 26<br>5187 ccpm | Object 34<br>4490 ccpm | Object 42<br>9814 ccpm | Object 50<br>6821 ccpm | Object 58<br>5374 ccpm | Object 66<br>4343 ccpm | Object 74<br>4054 ccpm | MAX<br>20613 ccpm |
| C | MAX<br>19536 ccpm | Object 3<br>13664 ccpm | Object 11<br>8407 ccpm | Object 19<br>5469 ccpm | Object 27<br>5442 ccpm | Object 35<br>4649 ccpm | Object 43<br>4994 ccpm | Object 51<br>4167 ccpm | Object 59<br>4529 ccpm | Object 67<br>3750 ccpm | Object 75<br>4236 ccpm | MAX<br>18772 ccpm |
| D | MIN<br>3665 ccpm | Object 4<br>5265 ccpm | Object 12<br>4176 ccpm | Object 20<br>4363 ccpm | Object 28<br>3820 ccpm | Object 36<br>4112 ccpm | Object 44<br>4155 ccpm | Object 52<br>4337 ccpm | Object 60<br>3913 ccpm | Object 68<br>3502 ccpm | Object 76<br>4075 ccpm | MIN<br>3620 ccpm |
| E | MIN<br>4308 ccpm | Object 5<br>4653 ccpm | Object 13<br>3695 ccpm | Object 21<br>4074 ccpm | Object 29<br>4822 ccpm | Object 37<br>3957 ccpm | Object 45<br>4131 ccpm | Object 53<br>4941 ccpm | Object 61<br>4430 ccpm | Object 69<br>3737 ccpm | Object 77<br>4131 ccpm | MIN<br>3739 ccpm |
| F | MIN<br>3617 ccpm | Object 6<br>6273 ccpm | Object 14<br>3850 ccpm | Object 22<br>4224 ccpm | Object 30<br>3804 ccpm | Object 38<br>5737 ccpm | Object 46<br>4455 ccpm | Object 54<br>4959 ccpm | Object 62<br>5728 ccpm | Object 70<br>4124 ccpm | Object 78<br>3803 ccpm | MIN<br>3283 ccpm |
| G | Ref.<br>13052 ccpm | Object 7<br>4568 ccpm | Object 15<br>4064 ccpm | Object 23<br>3918 ccpm | Object 31<br>4289 ccpm | Object 39<br>4512 ccpm | Object 47<br>4155 ccpm | Object 55<br>4767 ccpm | Object 63<br>4969 ccpm | Object 71<br>3909 ccpm | Object 79<br>3834 ccpm | Ref.<br>11294 ccpm |
| H | Ref.<br>13797 ccpm | Object 8<br>4147 ccpm | Object 16<br>3466 ccpm | Object 24<br>3866 ccpm | Object 32<br>3922 ccpm | Object 40<br>4137 ccpm | Object 48<br>6057 ccpm | Object 56<br>4163 ccpm | Object 64<br>4592 ccpm | Object 72<br>3806 ccpm | Object 80<br>4135 ccpm | Ref.<br>12645 ccpm |

Test von 80 Substanzen im NOS-Assay im 96 well-Mikrotiterplattenformat

Von den getesteten 80 Verbindungen zeigen 3 eine NOS-Inhibition (Positionen A2; C2 und B7; N = 1)

Der Mittelwert für die Referenzsubstanz 7-Nitroindazol (= interner Standard, 1 x $10^{-5}$M im Ansatz) lautet:

$$\bar{x} = 12\,697 \pm 1\,050 \text{ ccpm } (\bar{x} \pm SD; N=4)$$

Diese 7-Nitroindazol Konzentration ist bewußt so gewählt worden, daß sie sich im Bereich von ca. 50% Hemmung befindet.

Der Mittelwert der "Max"-Daten (ohne Enzym, Bezugswert für 100% [$^3$H]Arginin) stellt sich wie folgt dar:

$$\bar{x} = 19\,746 \pm 607 \text{ ccpm } (\bar{x} \pm SD; N=6)$$

Der Mittelwert der "Min"-Daten (ungehemmte Enzymreaktion) ergibt folgende Radioaktivität:

$$\bar{x} = 3\,158 \pm 334 \text{ ccpm } (\bar{x} \pm SD; N=6)$$

Als Gesamt-Meßstrecke stehen somit in diesem Beispiel 16 588 ccpm (19 746 - 3 158 ccpm) zur Verfügung.

**Beispiel 6:**

**Messung der IC50-Werte verschiedener Referenzsubstanzen mit einer der erfindungsgemäßen Methoden und der im Stand der Technik üblichen Messung des Citrullins mit Hilfe der Säulenchromatographie nach D.S. Bredt u. S.H. Snyder, Proc. Natl. Acad. Sci. 87, 682 (1990) im Vergleich.**

Die Messung des Citrullins mit Hilfe der Säulenchromatographie wurde nach der Beschreibung von D.S. Bredt u. S.H. Snyder, Proc. Natl. Acad. Sci. 87, 682-685 (1990) durchgeführt, wobei das Substrat, [$^3$H]-Arginin, im Ansatz in einer Konzentration von 50 nM vorlag und das Enzym isolierte nNOS aus Ratten-Cerebelli war, wie in Beispiel 3 beschrieben. Die Auftrennung erfolgte über Säulenchromatographie.

Das erfindungsgemäße Vorgehen entsprach dem Vorgehen nach Beispiel 4, wobei auch hier das [$^3$H]Substrat, [$^3$H]-Arginin, im Ansatz in einer Konzentration von 50 nM vorlag und das Enzym isolierte nNOS aus Ratten-Cerebelli war, wie in Beispiel 3 beschrieben. Die Auftrennung erfolgte erfindungsgemäß über 96-Well Kationenaustauscher Multititerplatten (MTP/Filter-MTP).

Als Testsubstanzen wurden die folgenden bekannten NOS-Inhibitoren zur Bestimmung der IC50-Werte in verschiedenen Konzentrationen in beide zu vergleichenden Tests eingesetzt:

- 7-Nitroindazol,
- 1-(2-Trifluormethylphenyl)imidazol (=TRIM),
- $N^G$-Monomethyl-L-arginin (=L-NMMA),
- N5-(1-Imino-3-butenyl)-L-ornithin (=Vinyl-L-NIO)
- N-[3-(Aminomethyl)benzyl]acetamidin (=1400W)
- N5-[imino(propylamino)methyl]-L-omithin (=N-Pr-L-Arg)

Die Ergebnisse sind der folgenden Tabelle 4 zu entnehmen.

Tabelle 4:

| | **IC50-Werte [µM]** | |
|---|---|---|
| **Substanz** | **Citrullin-Assay (nach Bredt u. Snyder (1990)** | **Erfindungsgemäßer Arginin-Assay gemäß Beispiel 4** |
| **7-Nitroindazol** | **5,23** | **4,27** |

Tabelle 4: (fortgesetzt)

| | IC50-Werte [µM] | |
|---|---|---|
| **Substanz** | **Citrullin-Assay (nach Bredt u. Snyder (1990)** | **Erfindungsgemäßer Arginin-Assay gemäß Beispiel 4** |
| **TRIM** | **>100** | **>100** |
| **L-NMMA** | **4,67** | **6,07** |
| **Vinyl-L-NIO** | **5,57** | **5,02** |
| **1400W** | **16,74** | **6,98** |
| **N-Pr-L-Arg** | **1,07** | **1,00** |

Wie aus Tabelle 4 ersichtlich liegen die mit den beiden unterschiedlichen Methoden gemessenen IC50-Werte sehr eng beieinander und unterscheiden sich bei den 6 Substanzen selbst im ungünstigsten Fall lediglich um den Faktor 2,4. Dies ist ein klarer Nachweis, daß beide Methoden für die Bestimmung des IC50 voll adäquat sind und damit das erfindungsgemäße Verfahren ein sehr valides Verfahren zur Bestimmung der NOS-Inhibition ist, das qualitativ mit der wegen der Säulenchromatographie sehr viel aufwendigeren bekannten Standardmethode, der Citrullin-Methode nach Bredt u. Snyder (1990), durchaus vergleichbar ist. Angesichts der Tatsache, daß das erfindungsgemäße Verfahren sehr viel einfacher, günstiger und sogar HTSfähig ist, liegt hier ein großer Vorteil des erfindungsgemäßen Vorgehens.

## Patentansprüche

**1.** Verfahren zur Messung der Aktivität der NO-Synthase mit folgenden Verfahrensschritten:

(a) Inkubation der NO-Synthase mit markiertem Arginin als Substrat in einem Reaktionsgefäß,
(b) Trennung des markierten Arginins von dem gegebenenfalls als Produkt der enzymatischen Reaktion entstandenen, markierten Citrullin, zu einem Zeitpunkt, zu dem die Konzentration an Citrullin ansteigt,
(c) Messung der Menge an jeweils abgetrenntem Arginin,

**dadurch gekennzeichnet, daß** die Trennung über eine Kationenaustausch-Filterplatten-Membran erfolgt.

**2.** Verfahren zur Identifizierung von NO-Synthase-Modulatoren mit folgenden Verfahrensschritten:

(a) Inkubation einer zu testenden Substanz mit NO-Synthase und markiertem Arginin als Substrat in mindestens einem Reaktionsgefäß,
(b) Trennung des markierten Arginins von dem gegebenenfalls als Produkt der enzymatischen Reaktion entstandenen, markierten Citrullin, zu einem Zeitpunkt, zu dem die Konzentration an Citrullin ansteigt,
(c) Messung der Menge an jeweils abgetrenntem Arginin, **dadurch gekennzeichnet, daß** die Trennung über eine Kationenaustausch-Filterplatten-Membran erfolgt.

**3.** Verfahren gemäß Anspruch 2, **dadurch gekennzeichnet, daß** das Verfahren zur Identifizierung eines Aktivators oder Inhibitors, vorzugsweise eines Inhibitors, der NO-Synthase dient.

**4.** Verfahren gemäß einem der Ansprüche 1 bis 3, **dadurch gekennzeichnet, daß** bei der Trennung gemäß Schritt (b) (wie in Anspruch 1 oder 2 beschrieben) aus dem Reaktionsgefäß Inkubationsmedium entfernt wird.

**5.** Verfahren gemäß Anspruch 4, **dadurch gekennzeichnet, daß** das Inkubationsmedium weitgehend, insbesondere fast vollständig, vorzugsweise vollständig, entfernt wird.

**6.** Verfahren gemäß einem der Ansprüche 4 oder 5, **dadurch gekennzeichnet, daß** das markierte Arginin von dem Inkubationsmedium getrennt wird.

**7.** Verfahren gemäß einem der Ansprüche 1 bis 6, **dadurch gekennzeichnet, daß** bei der Trennung gemäß Schritt (b) (wie in Anspruch 1 oder 2 beschrieben) zusätzlich die Cofaktoren bzw. Coenzyme, die NO-Synthase, NO und

die Folgeprodukte und/oder die NO-Synthase von dem markierten Arginin getrennt werden.

**8.** Verfahren gemäß einem der Ansprüche 1 bis 7, **dadurch gekennzeichnet, daß** die Filterplatten-Membranen Papierfilter oder Textilfilter sind oder aus sonstigen porösen oder durchbrochenen Festmaterialien, beispielsweise Metall, Glas oder Keramik, bestehen oder diese enthalten.

**9.** Verfahren gemäß Anspruch 8, **dadurch gekennzeichnet, daß** die funktionellen Gruppen für den Kationenaustausch an den Filterplatten-Membranen fixiert sind.

**10.** Verfahren gemäß einem der Ansprüche 8 oder 9, **dadurch gekennzeichnet, daß** die funktionellen Gruppen des Kationenaustauschers Phosphat-, Carbonat-, Phosphonat-, Sulfat- und/oder Sulfonat-Gruppen, vorzugsweise Phosphat-Gruppen, sind.

**11.** Verfahren gemäß einem der Ansprüche 1 bis 10, **dadurch gekennzeichnet, daß** die Filterplatten-Membranen Papierfilter einer Dicke von 10,00 mm bis 0,10 mm, vorzugsweise 1,00 mm bis 0,15 mm, insbesondere 0,50 mm bis 0,20 mm, vorzugsweise 0,23 mm sind.

**12.** Verfahren gemäß einem der Ansprüche 1 bis 11, **dadurch gekennzeichnet, daß** die Filterplatten-Membranen Kationenaustauscher mit einer Austauschkapazität von $\geq 1{,}0\ \mu Eq./cm^2$, insbesondere $\geq 10{,}0\ \mu Eq./cm^2$, vorzugsweise $\geq 18{,}0\ \mu Eq./cm^2$ sind.

**13.** Verfahren gemäß einem der Ansprüche 1 bis 12, **dadurch gekennzeichnet, daß** Schritt (a) (wie in Anspruch 1 oder 2 beschrieben) parallel in mehreren Reaktionsgefäßen annähernd gleichzeitig, vorzugsweise gleichzeitig, erfolgt und/oder Schritt b) (wie in Anspruch 1 oder 2 beschrieben) parallel in mehreren Reaktionsgefäßen annähernd gleichzeitig, vorzugsweise gleichzeitig, erfolgt.

**14.** Verfahren gemäß Anspruch 13, **dadurch gekennzeichnet, daß** die Reaktionsgefäße zusammenhängen, es sich insbesondere um "Wells" einer Mikrotiterplatte handelt.

**15.** Verfahren gemäß Anspruch 14, **dadurch gekennzeichnet, daß** die Mikrotiterplatte < 96 "Wells", vorzugsweise 24 oder 48 "Wells", oder ≥ 96 "Wells", insbesondere 96, 384, 864 oder 1536 "Wells", aufweist.

**16.** Verfahren gemäß einem der Ansprüche 1 bis 15, **dadurch gekennzeichnet, daß** die Reaktionsgefäße für Schritt (a) und/oder Schritt (b) (wie in Anspruch 1 oder 2 beschrieben) ein Volumen zur Aufnahme von ≤ 2 ml, insbesondere ≤ 1 ml, vorzugsweise ≤ 1ml, ≤ 800 µl, ≤ 350 µl, ≤ 300 µl, ≤ 250 µl, ≤ 200 µl, ≤ 150 µl, ≤ 100 µl, ≤ 50 µl, ≤ 30 µl oder ≤ 5 µl aufweisen.

**17.** Verfahren gemäß einem der Ansprüche 1 bis 16, **dadurch gekennzeichnet, daß** die Trennung gemäß Schritt (b) (wie in Anspruch 1 oder 2 beschrieben) durch Absaugen des Inkubationsmediums aus Schritt (a) durch die Filterplatten-Membran erfolgt.

**18.** Verfahren gemäß einem der Ansprüche 1 bis 17, **dadurch gekennzeichnet, daß** nach der Trennung gemäß Schritt (b) (wie in Anspruch 1 oder 2 beschrieben) die Fraktion, die das vom markierten Arginin getrennte markierte Citrullin enthält, verworfen wird.

**19.** Verfahren gemäß einem der Ansprüche 1 bis 18, **dadurch gekennzeichnet, daß** nach der Trennung gemäß Schritt (b) (wie in Anspruch 1 oder 2 beschrieben) das abgetrennte markierte Arginin auf oder in der Filterplattenmembran vorliegt.

**20.** Verfahren gemäß Anspruch 19, **dadurch gekennzeichnet, daß** das markierte Arginin auf oder in der Filterplatten-Membran gebunden ist.

**21.** Verfahren gemäß einem der Ansprüche 1 bis 20, **dadurch gekennzeichnet, daß** die Markierung die Messung des Arginins über Radioaktivität, Fluoreszenz, Lumineszenz oder Spektrums- bzw. Farbveränderung erlaubt.

**22.** Verfahren gemäß Anspruch 21, **dadurch gekennzeichnet, daß** die Messung des markierten Arginins im Szintillationszähler nach Zugabe von Szintillationsflüssigkeit auf die Filterplatten-Membran erfolgt.

**23.** Verfahren gemäß einem der Ansprüche 1 - 22, **dadurch gekennzeichnet, daß** Schritt (a) und Schritt (b) (wie in Anspruch 1 oder 2 beschrieben) nicht im gleichen Reaktionsgefäß stattfinden.

**24.** Verfahren gemäß einem der Ansprüche 1 - 23, **dadurch gekennzeichnet, daß** nach der Abtrennung gemäß Schritt (b) ein Waschschritt erfolgt, vorzugsweise ≤ 3 mal, insbesondere 1 mal, vorzugsweise mit Wasser oder Puffer, insbesondere mit einem Volumen ≤ 10 ml, insbesondere ≤ 5 ml, vorzugsweise ≤ 3 ml, insbesondere ≤ 2 ml.

**25.** Verfahren gemäß einem der Ansprüch 1 - 24, **dadurch gekennzeichnet, daß** als NO-Synthase rekombinante NO-Synthase verwendet wird.

**26.** Verfahren gemäß Anspruch 25, **dadurch gekennzeichnet, daß** die NO-Synthase subtypspezifisch ist, insbesondere eine human-, ratten- oder maus-spezifische eNOS, iNOS oder nNOS, insbesondere auch α- bzw. β-nNOS ist.

**27.** Verfahren gemäß einem der Ansprüche 1 - 24, **dadurch gekennzeichnet, daß** die eingesetzte NO-Synthase Teil eines Rohextrakts aus Wirbeltier-, vorzugsweise Säugetier-, Gewebe ist.

**28.** Verfahren gemäß Anspruch 27, **dadurch gekennzeichnet, daß** der Rohextrakt aus Nagetier gewonnen ist, und/oder aus dem ZNS stammt und/oder der aus Homogenat gewonnene Überstand ist, insbesondere der Überstand eines Homogenats aus Ratten- oder Maus-Cerebelium ist.

**29.** Verfahren gemäß einem der Ansprüche 1 - 28, **dadurch gekennzeichnet, daß** die eingesetzte Pröteinmenge im Inkubationsansatz gemäß Schritt (a) (wie in Anspruch 1 oder 2 beschrieben) pro $mm^2$ der in Schritt b) eingesetzten Filterplattenmembran maximal ≤ 5,0 µg, insbesondere ≤ 2,5 µg, vorzugsweise ≤ 1,0 µg, sein darf.

**30.** Verfahren gemäß einem der Ansprüche 1 bis 29, **dadurch gekennzeichnet, daß** dem Inkubationsansatz gemäß Schritt a) in Anspruch 1 oder 2 Cofaktoren bzw. Coenzyme hinzugefügt werden.

**31.** Verfahren gemäß Anspruch 30, **dadurch gekennzeichnet, daß** dem Inkubationsansatz NADPH hinzugefügt wird.

**32.** Verfahren gemäß Anspruch 30 oder 31, **dadurch gekennzeichnet, daß** dem Inkubationsansatz Calmodulin, $Ca^{2+}$, Tetrahydrobiopterin, FAD und/oder FMN hinzugefügt werden.

**33.** Verfahren gemäß einem der Ansprüche 27 oder 28, **dadurch gekennzeichnet, daß** dem Inkubationsansatz NADPH und $Ca^{2+}$ hinzugefügt werden.

**34.** Verfahren gemäß einem der Ansprüche 1 bis 33, **dadurch gekennzeichnet, daß** der Inkubationsansatz gemäß Schritt (a) (wie in Anspruch 1 oder 2 beschrieben) in Puffer als Inkubationsmedium angesetzt wird.

**35.** Verfahren gemäß einem der Ansprüche 1 bis 34, **dadurch gekennzeichnet, daß** die Inkubation in Schritt (a) (wie in Anspruch 1 oder 2 beschrieben) bei Raumtemperatur, 37° C, 25° C, 10° C oder 4° C, vorzugsweise Raumtemperatur, stattfindet.

**36.** Verfahren gemäß einem der Ansprüche 1 bis 35, **dadurch gekennzeichnet, daß** die Trennung gemäß Schritt (b) (wie in Anspruch 1 oder 2 beschrieben) 1- 600 min, insbesondere 3 - 120 min, vorzugsweise 5 - 60 min nach Beginn der Inkubation gemäß Schritt (a) stattfindet.

**37.** Verfahren gemäß einem der Ansprüche 1 bis 36, **dadurch gekennzeichnet, daß** es sich bei dem Verfahren um ein HTS (High-Throughput-Screening) Verfahren handelt.

**38.** Verfahren gemäß einem der Ansprüche 1 bis 37, **dadurch gekennzeichnet, daß** das Verfahren ein "signal-to-noise-ratio" von ≥ 4, insbesondere ≥ 5, vorzugsweise ≥ 6 oder ≥ 10 aufweist.

**39.** Verwendung einer Kationenaustausch-Filterplattenmembran in einem Verfahren, das zur Messung der Aktivität der NO-Synthase dient, wobei die Filterplattenmembran zur Trennung des markierten Arginins dient.

**40.** Verwendung gemäß Anspruch 39, **dadurch gekennzeichnet, daß** das Verfahren der Messung der Aktivität der NO-Synthase und/oder der Identifizierung von NO-Synthase-Modulatoren, vorzugsweise Aktivatoren oder Inhibitoren, dient.

**41.** Verwendung gemäß Anspruch 40, **dadurch gekennzeichnet, daß** es sich um ein Verfahren gemäß einem der Ansprüche 1 bis 38 handelt.

**42.** Verwendung gemäß einem der Ansprüche 39 bis 41, **dadurch gekennzeichnet, daß** es sich bei den Verfahren um ein Verfahren im Rahmen eines HTS (high-throughput-screening) handelt.

## Claims

**1.** Method of measuring the activity of NO synthase by means of the following process steps:

(a) Incubation of NO synthase with labelled arginine as the substrate in a reaction vessel
(b) Separation of the labelled arginine from the labelled citrulline possibly formed as a product of the enzymatic reaction at a moment when the concentration of citrulline is rising
(c) Measurement of the amount of arginine separated in each case,

**characterised in that** separation is achieved by means of a cation exchange filter plate membrane.

**2.** Method for identifying NO synthase modulators comprising the following process steps:

(a) incubation of a substance to be tested, with NO synthase and labelled arginine as the substrate in a reaction vessel,
(b) separation of the labelled arginine from the labelled citrulline possibly formed as a product of the enzymatic reaction, at a moment when the concentration of citrulline is rising,
(c) measurement of the amount of arginine separated in each case,

**characterised in that** separation is achieved by means of a cation exchange filter plate membrane.

**3.** Method according to claim 2, **characterised in that** the method for identifying an activator or inhibitor, preferably an inhibitor, aids NO synthase.

**4.** Method according to any one of claims 1 to 3, **characterised in that** in the separation according to step (b) (as described in claim 1 or 2), incubation medium is removed from the reaction vessel.

**5.** Method according to claim 4, **characterised in that** the incubation medium is substantially, in particular almost completely, preferably completely removed.

**6.** Method according to any one of claims 4 or 5, **characterised in that** the labelled arginine is separated from the incubation medium.

**7.** Method according to any one of claims 1 to 6, **characterised in that**, in the separation according to step (b) (as described in claim 1 or 2), additionally the cofactors or coenzymes, the NO synthase, NO and the secondary products and/or the NO synthase are separated from the labelled arginine.

**8.** Method according to any one of claims 1 to 7, **characterised in that** the filter plate membranes are paper filters or textile filters, or are made of or contain other porous or perforated solid materials, for example metal, glass or ceramics.

**9.** Method according to claim 8, **characterised in that** the functional groups for the cation exchange are fixed to the filter plate membranes.

**10.** Method according to any one of claims 8 or 9, **characterised in that** the functional groups of the cation exchanger are phosphate, carbonate, phosphonate, sulphate and/or sulphonate groups, preferably phosphate groups.

**11.** Method according to any one of claims 1 to 10, **characterised in that** the filter plate membranes are paper filters having a thickness of 10.00 mm to 0.10 mm, preferably 1.00 mm to 0.15 mm, in particular 0.50 mm to 0.20 mm, preferably 0.23 mm.

**12.** Method according to any one of claims 1 to 11, **characterised in that** the filter plate membranes are cation exchangers with an exchange capacity of ≥ 1.0 µEq./$cm^2$ in particular ≥ 10.0 µEq./$cm^2$, preferably ≥ 18.0 µEq./$cm^2$.

**13.** Method according to any one of claims 1 to 12, **characterised in that** step (a) (as described in claim 1 or 2) takes place in parallel in a plurality of reaction vessels almost simultaneously, preferably simultaneously, and/or step (b) (as described in claim 1 or 2) takes place in parallel in a plurality of reaction vessels almost simultaneously, preferably simultaneously.

**14.** Method according to claim 13, **characterised in that** the reaction vessels are connected, in particular they are wells of a microtitre plate.

**15.** Method according to claim 14, **characterised in that** the microtitre plate has < 96 wells, preferably 24 or 48 wells, or ≥ 96 wells, in particular 96, 384, 864 or 1536 wells.

**16.** Method according to any one of claims 1 to 15, **characterised in that** the reaction vessels for step (a) and/or step (b) (as described in claim 1 or 2) have a capacity to hold ≤ 2 ml, in particular ≤ 1 ml, preferably ≤ 1 ml, ≤ 800 µl, ≤ 350 µl, ≤ 300 µl, ≤ 250 µl, ≤ 150 µl, ≤ 100 µl, ≤ 50 µl, ≤ 30 µl, or ≤ 5 µl.

**17.** Method according to any one of claims 1 to 16, **characterised in that** the separation according to step (b) ( as described in claim 1 or 2) is effected by suction of the incubation medium from step (a) through the filter plate membranes.

**18.** Method according to any one of claims 1 to 17, **characterised in that** after the separation according to step (b) (as described in claim 1 or 2) the fraction which contains the labelled citrulline separated from the labelled arginine is disposed of.

**19.** Method according to any one of claims 1 to 18, **characterised in that** after the separation according to step b (as described in claim 1 or 2) the separated, labelled arginine is present on or in the filter plate membrane.

**20.** Method according to claim 19, **characterised in that** the labelled arginine is bound.

**21.** Method according to any one of claims 1 to 22, **characterised in that** the labelling permits measurement of the arginine by radioactivity, fluorescence, luminescence or spectrum or colour change.

**22.** Method according to claim 21, **characterised in that** measurement of the labelled arginine takes place in the scintillation counter after scintillation fluid has been added to the filter plate membrane.

**23.** Method according to any one of claims 1 to 22, **characterised in that** step (a) and step (b) (as described in claim 1 or 2) do not take place in the same reaction vessel.

**24.** Method according to any one of claims 1 to 23, **characterised in that** after the separation according to step (b) elution takes place, preferably ≤ 3 times, in particular once, preferably with water or buffer solution, in particular with a volume of ≤ 10 ml, in particular ≤ 5 ml, preferably ≤ 3 ml, in particular ≤ 2 ml.

**25.** Method according to any one of claims 1 to 24, **characterised in that** recombinant NO synthase is used as the NO synthase.

**26.** Method according to claim 27, **characterised in that** the NO synthase is subtype-specific, in particular human-, rat- or mouse-specific eNOS, iNOS or nNOS, in particular also α or β nNOS.

**27.** Method according to any one of claims 1 to 25, **characterised in that** the NO synthase used is part of a raw extract of vertebrate, preferably mammal tissue.

**28.** Method according to claim 27, **characterised in that** the raw extract is obtained from rodent, and/or comes from the CNS and/or the supernatant obtained from homogenate, in particular the supernatant of a homogenate of rat or mouse cerebellum.

**29.** Method according to any one of claims 1 to 28, **characterised in that** the amount of protein used in the incubation

mixture according to step (a) (as described in claim 1 or 2) is at most ≤ 5.0 µg, particularly < 2.5 µg, preferably ≤ 1.0 µg per $mm^2$ of the filter-plate membrane used in step b).

**30.** Method according to any one of claims 1 to 29, **characterised in that** cofactors or coenzymes are added to the incubation mixture according to step (a) in claim 1 or 2.

**31.** Method according to claim 30, **characterised in that** NADPH is added to the incubation mixture.

**32.** Method according to claim 30 or 31, **characterised in that** calmodulin, $Ca^{2+}$, tetrahydrobiopterin, FAD and/or FMN are added to the incubation mixture.

**33.** Method according to any one of claims 27 or 28, **characterised in that** NADPH and $Ca^{2+}$ are added to the incubation mixture.

**34.** Method according to any one of claims 1 to 33, **characterised in that** the incubation mixture according to step (a) (as described in claim 1 or 2) is placed in buffer as an incubation medium.

**35.** Method according to any one of claims 1 to 34, **characterised in that** the incubation in step (a) (as described in claim 1 or 2) takes place at room temperature, 37 °C, 25 °C, 10 °C or 4 °C, preferably room temperature.

**36.** Method according to any one of claims 1 to 35, **characterised in that** the separation according to step (b) (as described in claim 1 or 2) takes place 1 - 600 min, in particular 3 - 120 min, preferably 5 - 60 min after incubation in step (a) begins.

**37.** Method according to any one of claims 1 to 36, **characterised in that** it is suitable for HTS (high throughput screening).

**38.** Method according to any one of claims 1 to 37, **characterised in that** the method has a signal-to-noise ratio of ≥ 4, in particular ≥ 5, preferably ≥ 6 or ≥ 10.

**39.** Use of a cation exchange filter plate membrane in a method which is used for measuring the activity of NO synthase, the filter plate membrane being used to separate the labelled arginine.

**40.** Use according to claim 39, **characterised in that** the method is used to measure the activity of NO synthase and/or to identify NO synthase modulators. preferably activators or inhibitors.

**41.** Use according to claim 40 **characterised in that** it is a method according to any one of claims 1 to 40.

**42.** Use according to any one of the claims 39 to 41, **characterised in that** this method involves a method based on HTS (high throughput screening).

## Revendications

**1.** Méthode de mesure de l'activité de NO-synthase comprenant les étapes suivantes :

(a) incubation de la NO-synthase avec de l'arginine marquée utilisée comme substrat dans un récipient de réaction,
(b) séparation de l'arginine marquée de la citrulline marquée éventuellement formée comme produit de la réaction enzymatique à un moment où la concentration en citrulline croît,
(c) mesure de la quantité d'arginine séparée à chaque fois,

**caractérisée en ce que** la séparation est effectuée au moyen d'une membrane de plaque filtrante d'échange cationique.

**2.** Méthode d'identification de modulateurs de NO-synthase comprenant les étapes suivantes :

(a) incubation dans au moins un récipient de réaction, d'une substance à tester avec la NO-synthase et de

l'arginine marquée utilisée comme substrat,
(b) séparation de l'arginine marquée de la citrulline marquée éventuellement formée comme produit de la réaction enzymatique, à un moment où la concentration en citrulline croît,
(c) mesure de la quantité d'arginine séparée à chaque fois,

**caractérisée en ce que** la séparation est effectuée au moyen d'une membrane de plaque filtrante d'échange cationique.

**3.** Méthode suivant la revendication 2, **caractérisée en ce qu'**elle sert à l'identification d'un activateur ou d'un inhibiteur, avantageusement d'un inhibiteur, de la NO-synthase.

**4.** Méthode suivant l'une des revendications 1 à 3,
**caractérisée en ce que** le milieu d'incubation est retiré du récipient de réaction lors de la séparation selon l'étape (b) (comme décrit dans la revendication 1 ou 2).

**5.** Méthode suivant la revendication 4, **caractérisée en ce que** le milieu d'incubation est retiré en grande quantité, en particulier presque en totalité, notamment en totalité.

**6.** Méthode suivant l'une des revendications 4 ou 5,
**caractérisée en ce que** l'arginine marquée est séparée du milieu d'incubation.

**7.** Méthode suivant l'une des revendications 1 à 6,
**caractérisée en ce que** lors de la séparation selon l'étape (b) (comme décrit dans la revendication 1 ou 2), les cofacteurs ou coenzymes, la NO-synthase, l'oxyde NO et les dérivés et/ou la NO-synthase sont en outre séparés de l'arginine marquée.

**8.** Méthode suivant l'une des revendications 1 à 7,
**caractérisée en ce que** les membranes de plaques filtrantes sont des filtres de papier ou de matière textile ou sont formées d'autres matières poreuses ou matières solides perforées, par exemple métal, verre ou céramique, ou en contiennent.

**9.** Méthode suivant la revendication 8
**caractérisée en ce que** les groupes fonctionnels pour l'échange cationique sont fixés aux membranes de plaques filtrantes.

**10.** Méthode suivant l'une des revendications 8 ou 9,
**caractérisée en ce que** les groupes fonctionnels de l'échangeur cationique sont des groupes phosphate, carbonate, phosphonate, sulfate et/ou sulfonate, avantageusement des groupes phosphate.

**11.** Méthode suivant l'une des revendications 1 à 10,
**caractérisée en ce que** les membranes de plaques filtrantes sont des filtres en papier d'une épaisseur de 10,00 mm à 0,10 mm, avantageusement de 1,00 mm à 0,15 mm, en particulier de 0,50 mm à 0,20 mm, notamment de 0,23 mm.

**12.** Méthode suivant l'une des revendications 1 à 11,
**caractérisée en ce que** les membranes de plaques filtrantes sont des échangeurs cationiques ayant une capacité d'échange de $\geq 1,0\ \mu Eq/cm^2$, en particulier de $\geq 10,0\ \mu Eq/cm^2$, avantageusement de $\geq 18,0\ \mu Eq/cm^2$.

**13.** Méthode suivant l'une des revendications 1 à 12,
**caractérisée en ce que** l'étape (a) (comme décrit dans la revendication 1 ou 2) est conduite en parallèle dans plusieurs récipients de réaction à peu près en même temps, avantageusement en même temps et/ou l'étape (b) (comme décrit dans la revendication 1 ou 2) est conduite en parallèle dans plusieurs récipients de réaction à peu près en même temps, avantageusement en même temps.

**14.** Méthode suivant la revendication 13, caractérisée en que les récipients de réaction sont en rapport les uns avec les autres, ces récipients étant en particulier des "cupules" d'une plaque de microtitrage.

**15.** Méthode suivant la revendication 14, **caractérisée en ce que** la plaque de microtitrage présente moins de 96

"cupules", avantageusement 24 ou 48 "cupules", ou ≥ 96 "cupules", en particulier 96, 384, 864 ou 1536 "cupules".

16. Méthode suivant l'une des revendications 1 à 15,
**caractérisée en ce que** les récipients de réaction pour l'étape (a) et/ou pour l'étape (b) (comme décrit dans la revendication 1 ou 2) présentent un volume de réception de ≤ 2 ml, en particulier ≤ 1 ml, avantageusement ≤ 1 ml, ≤ 800 μl, ≤ 350 μl, ≤ 300 μl, ≤ 250 μl, ≤ 200 μl, ≤ 150 μl, ≤ 100 μl, ≤ 50 μl, ≤ 30 μl ou ≤ 5 μl.

17. Méthode suivant l'une des revendications 1 à 16,
**caractérisée en ce que** la séparation selon l'étape (b) (comme décrit dans la revendication 1 ou 2) est effectuée par "succion" du milieu d'incubation de l'étape (a) à travers la membrane de plaque filtrante.

18. Méthode suivant l'une des revendications 1 à 17,
**caractérisée en ce qu'**après la séparation selon l'étape (b) (comme décrit dans la revendication 1 ou 2), la fraction qui contient la citrulline marquée séparée de l'arginine marquée est jetée.

19. Méthode suivant l'une des revendications 1 à 18,
**caractérisée en ce qu'**après la séparation selon l'étape (b) (comme décrit dans la revendication 1 ou 2), l'arginine marquée séparée est présente sur ou dans la membrane de plaque filtrante.

20. Méthode suivant la revendication 19, **caractérisée en ce que** l'arginine marquée est liée sur ou dans la membrane de plaque filtrante.

21. Méthode suivant l'une des revendications 1 à 20,
**caractérisée en ce que** le marquage permet le dosage de l'arginine par radioactivité, fluorescence, luminescence ou par variation de spectre ou de couleur.

22. Méthode suivant la revendication 21, **caractérisée en ce que** le dosage de l'arginine marquée est effectué dans le compteur à scintillation après addition de liquide de scintillation sur la membrane de plaque filtrante.

23. Méthode suivant l'une des revendications 1 à 22,
**caractérisée en ce que** l'étape (a) et l'étape (b) (comme décrit dans la revendication 1 ou 2) n'ont pas lieu dans le même récipient de réaction.

24. Méthode suivant l'une des revendications 1 à 23,
**caractérisée en ce qu'**après la séparation selon l'étape (b), on effectue une étape de lavage, avantageusement ≤ 3 fois, en particulier 1 fois, avantageusement avec de l'eau ou un tampon, en particulier avec un volume ≤ 10 ml, en particulier ≤ 5 ml, avantageusement ≤ 3 ml, en particulier ≤ 2 ml.

25. Méthode suivant l'une des revendications 1 à 24,
**caractérisée en ce qu'**on utilise comme NO-synthase de la NO-synthase recombinée.

26. Méthode suivant la revendication 25, **caractérisée en ce que** la NO-synthase est spécifique de sous-types, étant en particulier une eNOS, iNOS ou nNOS spécifique de l'homme, du rat ou de la souris, et aussi en particulier une α- ou β-nNOS.

27. Méthode suivant l'une des revendications 1 à 24,
**caractérisée en ce que** la NO-synthase utilisée est une partie d'un extrait brut de tissu de vertébré, avantageusement de mammifère.

28. Méthode suivant la revendication 27, **caractérisée en ce que** l'extrait brut est obtenu à partir d'un rongeur et/ou provient du système nerveux central et/ou est le surnageant obtenu à partir d'un homogénéisat, en particulier le surnageant d'un homogénéisat de cervelet de rat ou de souris.

29. Méthode suivant l'une des revendications 1 à 28,
**caractérisée en ce que** la quantité utilisée de protéine dans le mélange d'incubation selon l'étape (a) (comme décrit dans la revendication 1 ou 2) par $mm^2$ de membrane de plaque filtrante utilisée dans l'étape (b) doit être au maximum ≤ 5,0 μg, en particulier ≤ 2,5 μg, avantageusement ≤ 1,0 μg.

**30.** Méthode suivant l'une des revendications 1 à 29,
**caractérisée en ce que** des cofacteurs ou des coenzymes sont ajoutés au mélange d'incubation selon l'étape (a) dans la revendication 1 ou 2.

**31.** Méthode suivant la revendication 30, **caractérisée en ce que** du NADPH est ajouté au mélange d'incubation.

**32.** Méthode suivant la revendication 30 ou 31,
**caractérisée en ce que** le mélange d'incubation est additionné de calmoduline, de $Ca^{2+}$, de tétrahydrobioptérine, de FAD et/ou de FMN.

**33.** Méthode suivant l'une des revendications 27 ou 28,
**caractérisée en ce que** le mélange d'incubation est additionné de NADPH ou de $Ca^{2+}$.

**34.** Méthode suivant l'une des revendications 1 à 33,
**caractérisée en ce que** le mélange d'incubation selon l'étape (a) (comme décrit dans la revendication 1 ou 2) est établi dans un tampon utilisé comme milieu d'incubation.

**35.** Méthode suivant l'une des revendications 1 à 34,
**caractérisée en ce que** l'incubation dans l'étape (a) (comme décrit dans la revendication 1 ou 2) a lieu à la température ambiante, à 37°C, à 25°C, à 10°C ou à 4°C, avantageusement à la température ambiante.

**36.** Méthode suivant l'une des revendications 1 à 35,
**caractérisée en ce que** la séparation selon l'étape (b) (comme décrit dans la revendication 1 ou 2) a lieu 1 à 600 minutes, en particulier 3 à 120 minutes, avantageusement 5 à 60 minutes après le début de l'incubation selon l'étape (a).

**37.** Méthode suivant l'une des revendications 1 à 36,
**caractérisée en ce qu'**elle est une méthode de criblage HTS (high-throughput-screening).

**38.** Méthode suivant l'une des revendications 1 à 37,
**caractérisée en ce qu'**elle présente un "signal-to-noise-ratio" $\geq 4$, en particulier $\geq 5$, avantageusement $\geq 6$ ou $\geq 10$.

**39.** Utilisation d'une membrane de plaque filtrante d'échange cationique dans une méthode qui sert à la mesure de l'activité de la NO-synthase, la membrane de plaque filtrante servant à la séparation de l'arginine marquée.

**40.** Utilisation suivant la revendication 39,
**caractérisée en ce que** la méthode sert à la mesure de l'activité de la NO-synthase et/ou à l'identification de modulateurs de NO-synthase, avantageusement d'activateurs ou d'inhibiteurs.

**41.** Utilisation suivant la revendication 40,
**caractérisée en ce que** la méthode est une méthode suivant l'une des revendications 1 à 38.

**42.** Utilisation suivant l'une des revendications 39 à 41, **caractérisée en ce que** la méthode consiste en une méthode dans le cadre d'un criblage HTS (high-throughput-screening).

Fig. 1)

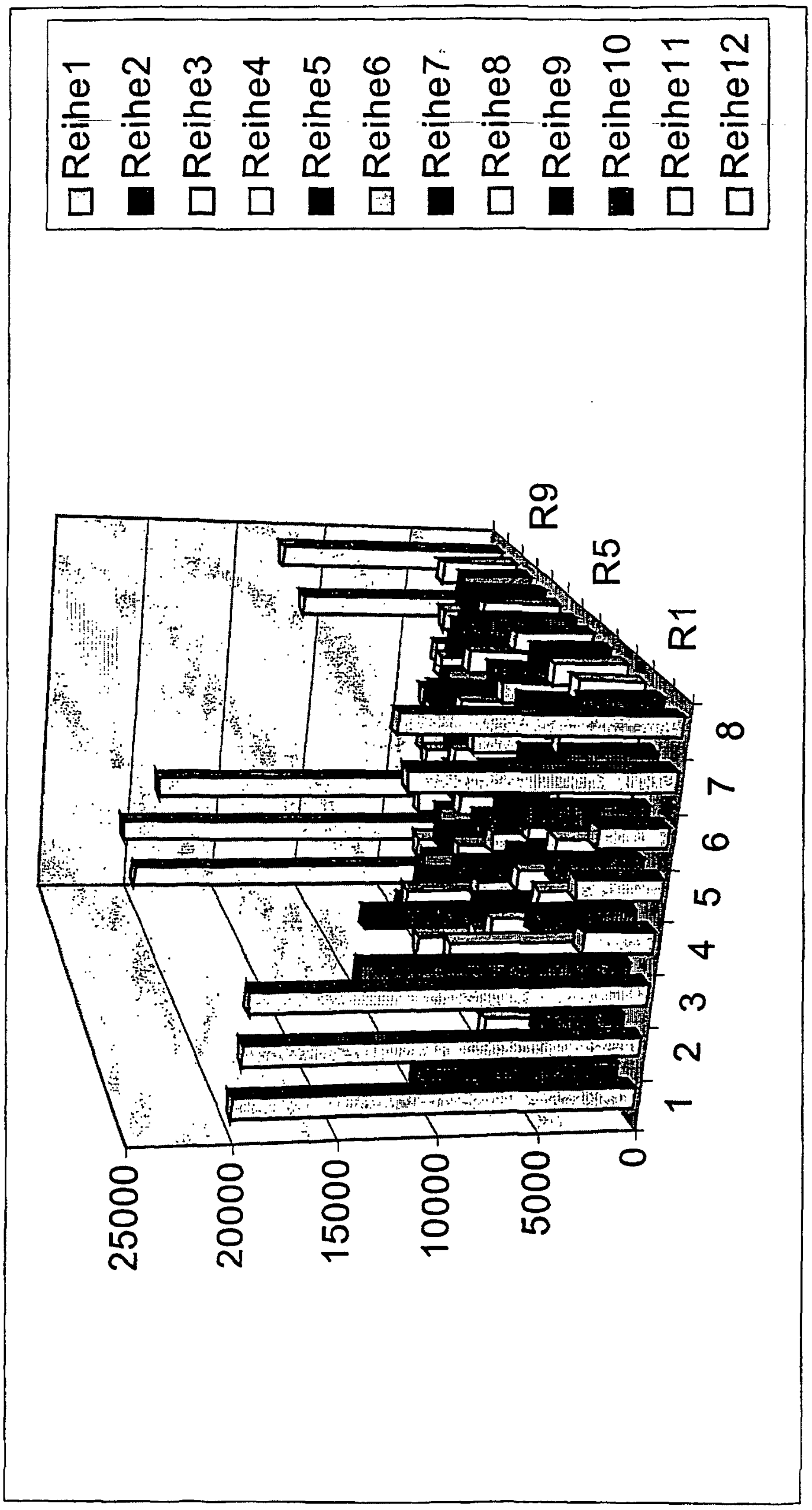
25000
20000
15000
10000
5000
0
1
2
3
4
5
6
7
8
R1
R5
R9
Reihe1
Reihe2
Reihe3
Reihe4
Reihe5
Reihe6
Reihe7
Reihe8
Reihe9
Reihe10
Reihe11
Reihe12